(19) Europäisches Patentamt
European Patent Office
Office européen des brevets

(11) **EP 4 129 970 A1**

(12) **EUROPEAN PATENT APPLICATION**
published in accordance with Art. 153(4) EPC

(43) Date of publication:
**08.02.2023 Bulletin 2023/06**

(21) Application number: **21776665.8**

(22) Date of filing: **22.03.2021**

(51) International Patent Classification (IPC):
***C07C 51/47*** (1980.01)    ***C07C 53/08*** (1968.09)

(52) Cooperative Patent Classification (CPC):
**C07C 51/47; C07C 53/08**

(86) International application number:
**PCT/JP2021/011666**

(87) International publication number:
**WO 2021/193511 (30.09.2021 Gazette 2021/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.03.2020   JP 2020056079**

(71) Applicant: **Daicel Corporation**
**Osaka 530-0011 (JP)**

(72) Inventors:
• **KIKUCHI, Yasuhiro**
  **Tokyo 108-8230 (JP)**
• **MITOME, Takahito**
  **Tokyo 108-8230 (JP)**
• **FUKUI, Naoyuki**
  **Tokyo 108-8230 (JP)**
• **TAKEDA, Kazuhito**
  **Tokyo 108-8230 (JP)**

(74) Representative: **Grünecker Patent- und
Rechtsanwälte
PartG mbB
Leopoldstraße 4
80802 München (DE)**

(54) **METHOD FOR PRODUCING PURIFIED ACETIC ACID**

(57)    Provided is a method for producing purified acetic acid with a good hue. A method for producing purified acetic acid, including treating acetic acid having a poor hue with a synthetic adsorbent to obtain acetic acid with an improved hue. The acetic acid to be treated is, for example, acetic acid having an absorbance of 0.01 or greater at a wavelength of 430 nm. The acetic acid to be treated may be acetic acid recovered from an acetic acid-containing solution that is discharged in a cellulose acetate production process. The synthetic adsorbent may have a cation exchange group on a resin surface. The synthetic adsorbent may have a pore structure.

EP 4 129 970 A1

**Description**

Technical Field

The present disclosure relates to a method for producing purified acetic acid, particularly a method for producing purified acetic acid with a good hue. The present application claims priority to JP 2020-056079 filed in Japan on March 26, 2020, the content of which is incorporated herein.

Background Art

Cellulose acetate is used in various applications such as cigarette filter tow, fiber, photographic film, and artificial kidney. As a method for producing cellulose acetate, there is known a method of reacting a raw material pulp or cellulose (hereinafter referred to as "raw material pulp or the like") with an acetylating agent in an aqueous acetic acid solution. However, in this method, an acetic acid-containing solution (hereinafter referred to as "raw acid") having an acetic acid concentration from 20 to 50 wt.%, in which a slight amount of cellulose acetate is dissolved, is discharged.

A common method for recovering acetic acid from this raw acid is extraction, and various extractants have been proposed and used (e.g., Patent Documents 1 and 2). The recovered acetic acid is reused in the production of cellulose acetate.

Citation List

Patent Document

Patent Document 1: JP 59-7131 A
Patent Document 2: JP 60-174743 A

Summary of Invention

Technical Problem

During a known production process of cellulose acetate, a hue deterioration-causing substance is produced as a byproduct from a raw material pulp or the like in a reactor (this substance is assumed to be composed mainly of a low molecular substance obtained by thermal decomposition of glucose originating from the raw material pulp or the like, for example, a substance in which two to three glucose molecule units are bound). This hue deterioration-causing substance is removed, for example, when acetic acid is extracted from the raw acid with an extractant and then the acetic acid, the extractant and water are separated by evaporation, but it is difficult to completely remove the hue deterioration-causing substance by entrainment. Therefore, in distillation which is a final step of an acetic acid recovery system, a hue deteriorating substance (this is assumed to be composed of a glucose decomposition product having a double bond, and the double bond site is assumed to contribute to coloration) is generated in a distillation column by heating.

The cellulose acetate produced using, as a raw material, acetic acid containing the hue deteriorating substance has a reduced quality due to coloration. In addition, when a large amount of hue deterioration-causing substance is contained in the raw material pulp, a large amount of hue deteriorating substance is also produced as a byproduct, and hue of the acetic acid recovered is also deteriorated. Therefore, an object of the present disclosure is to provide a method for producing purified acetic acid having a good hue.

Solution to Problem

As a result of diligent research to solve the problem described above, the present inventors have found that purified acetic acid with a good hue is obtained by contacting, with a synthetic adsorbent, for example, acetic acid with a poor hue which is obtained, through extraction, evaporation, and distillation, from a raw acid discharged in a cellulose acetate production process. The present disclosure has been completed based on these findings.

That is, the present disclosure provides a method for producing purified acetic acid, including treating acetic acid having a poor hue with a synthetic adsorbent to obtain acetic acid with an improved hue.

For example, the acetic acid to be treated has an absorbance of 0.01 or greater at a wavelength of 430 nm.

The acetic acid to be treated may be acetic acid recovered from an acetic acid-containing solution that is discharged in a cellulose acetate production process.

The synthetic adsorbent may have a cation exchange group on a resin surface.

Furthermore, the synthetic adsorbent may have a pore structure, and the pore structure may be a binary pore structure.

Advantageous Effects of Invention

According to the production method according to an embodiment of the present disclosure, it is possible to efficiently adsorb and remove the hue deteriorating substance contained in the acetic acid by the synthetic adsorbent, thus making it possible to industrially efficiently produce purified acetic acid with a good hue.

When the acetic acid to be treated is acetic acid recovered from the acetic acid-containing solution discharged in the cellulose acetate production process, the hue deteriorating substance contained in the acetic acid can be adsorbed and removed. Therefore, when the obtained purified acetic acid is recycled to the cellulose acetate production process, the hue of the cellulose acetate is also improved, and a high-quality cellulose acetate can be obtained.

Brief Description of Drawings

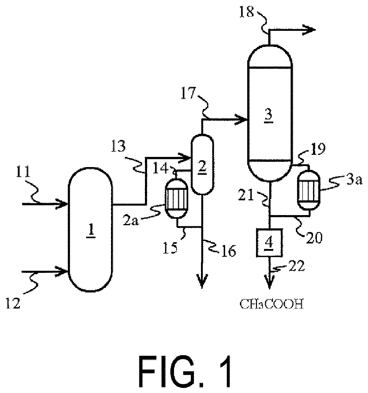

FIG. 1 is a schematic flow chart illustrating one embodiment of a method for producing purified acetic acid according to an embodiment of the present disclosure.

Description of Embodiments

In the method for producing purified acetic acid according to an embodiment of the present disclosure, acetic acid with an improved hue is obtained by treating acetic acid having a poor hue with a synthetic adsorbent.

(Acetic acid to be treated)

In the present disclosure, examples of acetic acid to be treated (acetic acid to be treated with a synthetic adsorbent) include acetic acid having an absorbance, at a wavelength of 430 nm, of 0.01 or greater, and preferably 0.02 or greater (for example, 0.025 or greater, especially greater than 0.03). Specific examples of the acetic acid to be treated include acetic acid recovered from an acetic acid-containing solution (raw acid) discharged in a cellulose acetate production process. The acetic acid recovered from the raw acid typically contains a hue deteriorating substance (such as a glucose decomposition product having a double bond), into which a hue deterioration-causing substance produced as a byproduct from a raw material pulp or the like (which is assumed to be composed mainly of a low molecular substance obtained by thermal decomposition of glucose originating from the raw material pulp or the like, for example, a substance in which two to three glucose molecule units are bound) is converted by heat at the time of distillation or the like. When such acetic acid is treated with the synthetic adsorbent, the hue deteriorating substance is adsorbed by the synthetic adsorbent, and purified acetic acid with an excellent hue can be obtained. Note that, in the present specification, the absorbance is a value as measured by an absorbance measurement apparatus using an optical path length of 10 mm and a pure acetic acid as a reference cell content liquid.

(Synthetic adsorbent)

In the present disclosure, a synthetic adsorbent is used as a treatment agent for acetic acid with a poor hue. Examples of the synthetic adsorbent include, but is not limited to, polystyrene-based synthetic adsorbents and polymethacrylate-based synthetic adsorbents. The synthetic adsorbents include synthetic adsorbents having an ion exchange group on their surface, and synthetic adsorbents that do not have an ion exchange group on their surface. Also, the synthetic adsorbents include synthetic adsorbents having a pore structure (porous structure), and synthetic adsorbents that do not have a pore structure (porous structure). By using a synthetic adsorbent as a treatment agent for acetic acid with a poor hue, the hue deteriorating substance in the acetic acid is efficiently adsorbed and removed, due to hydrophobic attraction, interaction between dipoles, hydrogen bond, ion exchange action, or interaction thereof, depending on surface characteristics of the synthetic adsorbent.

Among the synthetic adsorbents, a polystyrene-based synthetic adsorbent is preferable from the perspective of hue improvement effect of acetic acid. Examples of the polystyrene-based synthetic adsorbent include (a) a synthetic adsorbent containing a styrene-based polymer as a resin matrix and (b) a synthetic adsorbent containing a styrene-based crosslinked copolymer as a resin matrix. The synthetic adsorbents (a) and (b) can also be used in combination.

Examples of the styrene-based polymer constituting the resin matrix of the (a) synthetic adsorbent include polymers of styrene-based monomers such as styrene, ethylstyrene, and methylstyrene. Among them, a styrene polymer (polystyrene) is preferred. These resin matrices have strong hydrophobicity. The (a) synthetic adsorbent may have a cation exchange group described later on its surface.

Examples of the styrene-based crosslinked copolymer constituting the resin matrix of the (b) synthetic adsorbent include crosslinked copolymers of a styrene-based monomer such as styrene, ethylstyrene, or methylstyrene and a crosslinking agent such as divinylbenzene or trivinylbenzene. Among these, a crosslinked copolymer of styrene and divinylbenzene is preferred. These resin matrices have strong hydrophobicity. The (b) synthetic adsorbent may have a cation exchange group described later on its surface.

Examples of the ion exchange group that may be included on the surface of the synthetic adsorbent include cation exchange groups such as a carboxy group, a sulfonate group, and a phosphonate group, and anion exchange groups such as an amino group and a quaternary ammonium group. Among these, a cation exchange group, particularly a sulfonate group or a strongly acidic cation exchange group having an acidity equal to or higher than that of a sulfonate group, is preferred. Introducion of the ion exchange group such as the cation exchange group enhances the hydrophilicity of the synthetic adsorbent, and an adsorption effect can be effectively exhibited due to an interaction of ion exchange and hydrophobic attraction.

The pore structure that may be possessed by the synthetic adsorbent preferably has, for example, one or more of an average pore size of 10 nm or less (e.g., 9 nm, and preferably 8 nm) or an average pore size of 20 nm or greater (e.g., 25 nm or greater, and preferably 30 nm or greater). When the average pore size of the synthetic adsorbent is 10 nm or less, it is possible to impart a specific surface area sufficient to effectively adsorb and retain the hue deteriorating substance to the resin matrix. When the average pore size of the synthetic adsorbent is 20 nm or greater, acetic acid tends to penetrate and diffuse, an adsorption capacity is increased, and the hue deteriorating substance can be effectively removed. From the above viewpoint, a lower limit of the average pore size is preferably 2 nm, and more preferably 5 nm. An upper limit of the average pore size is preferably 300 nm, and more preferably 100 nm.

The synthetic adsorbent more preferably has a binary pore structure including large pores and small pores as described above in combination, from the perspective of more effectively removing the hue deteriorating substance. For example, the synthetic adsorbent preferably has a binary pore structure including small pores having an average pore size of 10 nm or less (e.g., from 2 to 10 nm) and large pores having an average pore size of 20 nm (e.g., from 20 to 100 nm). A method for measuring the average pore size of the synthetic adsorbent can be measured as appropriate by a known method. For example, the average pore size of the synthetic adsorbent can be measured using a gas adsorption method, a small angle X-ray scattering method or a mercury intrusion method.

A specific surface area of the synthetic adsorbent is, for example, from 300 $m^2/g$ to 1,500 $m^2/g$, and preferably from 400 $m^2/g$ to 1,200 $m^2/g$. When the specific surface area is within the range as described above, an adsorption surface for adsorbing the hue deteriorating substance in the acetic acid is sufficiently provided. Furthermore, the pore volume of the synthetic adsorbent can be adjusted as appropriate depending on an amount of the hue deteriorating substance contained in the acetic acid, and is, for example, from 0.1 to 2.0 mL/g, and preferably from 0.2 to 1.7 mL/g. The specific surface area and pore volume of the synthetic adsorbent can be measured as appropriate by a known method. For example, they can be measured using a gas adsorption BET method (one-point method, a multi-point method), or the like.

A form of the synthetic adsorbent is not particularly limited, and may include a powdery material, a granular material, a particulate material, and a structure. Also, a common shape of the synthetic adsorbent is amorphous, spherical, or the like, and a spherical shape is preferable, and an average particle size thereof is from 200 to 1,500 μm, and preferably from 300 to 1,200 μm. Furthermore, from the perspective of obtaining uniform particles, a uniformity coefficient of the synthetic adsorbent is preferably 1.6 or less. The average particle size of the synthetic adsorbent can be measured as appropriate by a known method. For example, it can be measured using a laser diffraction particle size distribution measurement apparatus or a sieving method.

A moisture content of the synthetic adsorbent is, for example, from 45 to 75%, and preferably from 50 to 70% . When the moisture content of the synthetic adsorbent is within the range described above, there is an advantage of a smaller volume change due to swelling and contraction at the time of passage of acetic acid, and easiness to handle.

(Adsorption treatment)

When the acetic acid to be treated (acetic acid with a poor hue) is treated with the synthetic adsorbent, an amount of the synthetic adsorbent used is, for example, from 0.1 to 15 parts by weight, preferably from 0.1 to 10 parts by weight, and more preferably from 0.1 to 5 parts by weight with respect to 100 parts by weight of the acetic acid, for example, in a case of treatment by a batch method, from the perspective of efficiently removing the hue deteriorating substance. The amount of the synthetic adsorbent used can be adjusted as appropriate depending on the amount of the hue deteriorating substance contained in the acetic acid. Note that the synthetic adsorbent after use can be repeatedly used by regeneration with an acid (such as hydrochloric acid), an alkali (such as sodium hydroxide) or the like, or by regeneration through steam washing or elution with an organic solvent.

A method of bringing the acetic acid into contact with the synthetic adsorbent is not particularly limited. Examples of the method may include a batch method in which the acetic acid, the synthetic adsorbent, and, as necessary, a solvent are added to a reaction vessel and stirred, and a method (continuous method) in which the acetic acid is caused to pass through a column packed with the synthetic adsorbent. Note that impurities contained in the acetic acid may be removed, in advance, using diatomaceous earth, activated carbon, or the like.

A temperature at which the acetic acid is brought into contact with the synthetic adsorbent is typically performed at approximately from 5 to 70°C, and preferably from 15 to 40°C, regardless of the contact method. When the contact temperature is within the range described above, a passage velocity is appropriate, and work efficiency is excellent. Also, a contact time between the acetic acid and the synthetic adsorbent varies depending on the amount of the hue deteriorating substance in the acetic acid, the type of the synthetic adsorbent, and temperature conditions, and is typically 20 minutes or longer. The contact between the acetic acid and the synthetic adsorbent can be performed under atmospheric pressure, under increased pressure, or under reduced pressure.

A space velocity (SV) when acetic acid is caused to pass through the column packed with synthetic adsorbent is, for example, SV = from 0.5 to 5 (1/Hr), and preferably SV = from 1 to 2 (1/Hr). Here, SV is a unit indicating in an amount how many times the amount of acetic acid is passed per hour to the volume of the synthetic adsorbent, and can be determined from the following equation (1).

$$\text{SV} = \text{flow rate (L/Hr)/packing amount of synthetic adsorbent (L)} \bullet\bullet\bullet\bullet\bullet \text{ (1)}$$

As the synthetic adsorbent, a commercially available product can be used. The commercially available product of the (a) synthetic adsorbent may include, for example, the trade name "T-C150" available from Purolite K.K. The commercially available product of the (b) synthetic adsorbent may include, for example, the trade name "MN200" and the trade name "MN502" available from Purolite K.K.

(One embodiment)

FIG. 1 is a schematic flow chart illustrating one embodiment of a method for producing purified acetic acid according to an embodiment of the present disclosure. In this embodiment, acetic acid recovered from an acetic acid-containing solution (raw acid) discharged in a cellulose acetate production process is treated with a synthetic adsorbent. An apparatus for producing purified acetic acid according to this production flow includes an extraction column 1, an evaporator 2, a distillation column 3, an adsorption apparatus 4, reboilers 2a and 3a, and lines 11 to 22.

First, the raw acid is brought into contact with an extractant, and is separated into an extraction liquid phase mainly containing acetic acid and the extractant and an extraction residual phase mainly containing water (extraction). In FIG. 1, the raw acid is input into the extraction column 1 through the line 11. An acetic acid concentration of the raw acid is typically from 20 to 50 wt.%. From the raw acid, impurities may be removed, in advance, using diatomaceous earth, activated carbon, or the like.

The extractant is input through the line 12 into the extraction column 1. The extractant is not particularly limited, and a solvent providing high level of acetic acid dissolution and having a low solubility in water is preferably used. For example, an aromatic hydrocarbon such as benzene, toluene, or xylene; an aliphatic hydrocarbon such as n-hexane, cyclohexane, heptane, octane, or nonane; an ester such as ethyl acetate, n-amyl acetate, cyclohexyl acetate, isoamyl propionate, or methyl benzoate; a halide such as chloroform, carbon tetrachloride, or chlorobenzene; or an ether compound such as 1,2-dimethoxyethane or dimethyl ether is preferred. One of these extractants can be used alone, or two or more thereof can be used in combination. Among the extractants, a mixed solvent of an ester such as ethyl acetate (particularly preferably, acetic acid ester) and an aromatic hydrocarbon such as benzene is preferred.

As the extraction column 1, there can be used an extraction apparatus of a commonly used form, for example, a mixer-settler type extraction column, a porous plate type, a packed column type, a baffle column type, a vibrating porous plate type, a stirring-mixing type, a packed pulse type, a centrifugal extraction type, or the like. The contact between the raw acid and the extractant can be performed under atmospheric pressure, under increased pressure, or under reduced pressure.

The extraction liquid phase obtained in this step mainly contains acetic acid and the extractant, and a water content is, for example, 15 wt.% or less, and preferably 10 wt.% or less. A cellulose acetate content is 200 ppm or less (preferably 150 ppm or less). On the other hand, the extraction residual phase mainly contains water and soluble cellulose acetate, and a content of acetic acid is, for example, 0.5 wt.% or less, and preferably 0.1 wt.% or less.

The thus obtained extraction liquid phase is input into the evaporator 2 through the line 13 and separated, by evaporation, into vapor containing acetic acid, the extractant, and water and an evaporation residual liquid containing soluble cellulose acetate (evaporation). In this step, the separated soluble cellulose acetate is discharged out of the system through the line 16. In this step, the cellulose acetate dissolved in the extraction liquid phase can be separated and removed.

In this step, the extraction liquid phase may be separated into vapor containing acetic acid, the extractant, and water and the evaporation residual liquid containing soluble cellulose acetate, for example, by subjecting the extraction liquid phase to heat treatment. A heat treatment temperature is, for example, from 80 to 100°C. The heat treatment can be performed under atmospheric pressure, under increased pressure, or under reduced pressure. This step may be composed of a single step or a plurality of steps in combination. As the evaporator 2, a known evaporator can be used, and a distillation tank of a distillation column form may be used.

Next, acetic acid and the extractant are separated by utilizing a difference in boiling point, the extractant is distilled off, and a high-purity acetic acid is obtained as a purified liquid (distillation). In this step, the extraction liquid phase may be charged into the distillation column 3 in a liquid phase state (not illustrated), and the vapor may be charged into the distillation column 3 through the line 17.

When the extraction liquid phase or the vapor is charged into the distillation column 3, most of the extractant contained in the extraction liquid phase or the vapor is moved from a top of the column via the line 18, cooled with a cooler (not illustrated), and input into a tank (not illustrated). An upper layer liquid, of liquids input into the tank, mainly contains the extractant, and is discharged out of the system. This liquid can again be reused as an extractant to be input into the extraction column 1. A lower layer liquid in the tank mainly contains water, and is discharged out of the system. On the other hand, acetic acid is obtained from a column bottom of the distillation column 3 via the line 21.

Examples of the distillation column 3 may include a plate column and a packed column. A column top temperature is, for example, from 20 to 120°C, preferably from 30 to 100°C, and more preferably from 40 to 80°C. Furthermore, pressure in the distillation column 3 is, for example, from 0.01 to 0.5 MPa, preferably from 0.05 to 0.4 MPa, and even more preferably from 0.1 to 0.2 MPa as absolute pressure. The distillation may be composed of a single step or a plurality of steps in combination.

When the charge into the distillation column 3 is vapor charge and liquid phase charge in combination, it is possible to improve separation efficiency as compared with a case where all the charge into the distillation column 3 is vapor charge. Therefore, a reflux ratio in the distillation column 3 can be set to be low, and is, for example, in the range from 0.5 to 1.0, and preferably in the range from 0.6 to 0.9.

Through the distillation, a high-purity acetic acid is obtained as a purified liquid through the line 21. An acetic acid concentration of a distillate is, for example, 0.02 wt.% or less, preferably 0.01 wt.% or less, and more preferably 0.005 wt.% or less. The acetic acid concentration of the distillate can be controlled by adjusting the reflux ratio in the distillation column 3.

Then, the acetic acid obtained as a purified liquid in the distillation is brought into contact with the synthetic adsorbent (adsorption). This step can be performed by inputting the acetic acid into the adsorption apparatus 4 through the line 21. As the synthetic adsorbent, those described above can be used. Also, a method, conditions, and the like for contact between the acetic acid and the synthetic adsorbent are as described above.

By performing the adsorption, the hue deteriorating substance can be efficiently adsorbed and removed, and a highly purified acetic acid (hereinafter referred to as "purified acetic acid") can be obtained. According to the method for producing purified acetic acid according to an embodiment of the present disclosure, it is possible to produce purified acetic acid having a hue of acetic acid of 0.03 or less (e.g., 0.028 or less, preferably 0.025 or less, and more preferably 0.02 or less) as an absorbance at a wavelength of 430 nm. The purified acetic acid can again be incorporated in the cellulose acetate production process, and can also be charged into a reactor together with the raw material pulp or the like. This makes it possible to suppress coloration of cellulose acetate, and thus a hue of the cellulose acetate can also be improved.

Each aspect disclosed in the present specification can be combined with any other feature disclosed herein. Note that each of the configurations, combinations thereof, and the like in each of the embodiments are an example, and various additions, omissions, and other changes may be made as appropriate without departing from the spirit of the present disclosure. The present disclosure is not limited by the embodiments and is limited only by the claims.

Examples

Hereinafter, the present disclosure will be more specifically described with reference to examples.

Example 1

As the synthetic adsorbent, a product under the trade name "MN502" available from Purolite K.K. (resin matrix: polystyrene-divinylbenzene crosslinked polymer, a strongly acidic cation exchange group introduced, a binary pore structure including pores having an average pore size of 10 nm or less and pores having an average pore size of 65 nm) was taken in an appropriate amount, and washed several times with water and acetic acid. Thereafter, 0.1 g of the synthetic adsorbent was added to 10 g of acetic acid recovered from an acetic acid-containing solution (raw acid) discharged in a cellulose acetate production process (purified liquid obtained through the extraction, evaporation, and distillation) (purity: 99.9 wt.%), and a test liquid was prepared. The test liquid was stirred at room temperature for 6 hours. The test liquid after stirring was filtered through a membrane filter (trade name "Membrane Filter", pore size: 1 μm, material: PTFE, available from ADVANTEC), and a filtrate was obtained. An absorbance of the obtained filtrate at a wavelength of 430 nm was measured using a UV absorbance measurement apparatus (under the trade name "UV-3900H", available from Hitachi, Ltd.) (optical path length: 10 mm, reference cell content liquid: pure acetic acid). The result shows that the absorbance was 0.018.

Example 2

The absorbance at a wavelength of 430 nm was measured in the same manner as in Example 1 except that a product under the trade name "MN200" available from Purolite K.K. (resin matrix: polystyrene-divinylbenzene crosslinked polymer, no cation exchange group, a binary pore structure including pores having an average pore size of 10 nm or less and pores having an average pore size of 70 nm) was used as the synthetic adsorbent. The result shows that the absorbance was 0.027.

Example 3

The absorbance at a wavelength of 430 nm was measured in the same manner as in Example 1, except that a product under the trade name "T-C150" available from Purolite K.K. (resin matrix: polystyrene, cation exchange group introduced, no pore structure) was used as the synthetic adsorbent. The result shows that the absorbance was 0.023.

Comparative Example 1

As a blank, the absorbance at a wavelength of 430 nm of the purified acetic acid (purity: 99.9 wt.%) used as the raw material in Example 1 was measured. The result shows that the absorbance was 0.032.

(Discussion of results)

Comparative Example 1 is the blank. In the comparison between Examples 1 and 2, the resin matrix, styrene pore structure (binary pore structure) and pore size were substantially equivalent, but Example 1 (synthetic adsorbent with a cation exchange group) showed a better hue improvement effect. Example 3, which is an example involving the use of a synthetic adsorbent containing a styrene-based polymer as the resin matrix and a cation exchange group introduced to a resin surface, and having no pore structure, was higher in absorbance than Example 1.

As a summary of the above, configurations and variations of the present disclosure are described below.

[1] A method for producing purified acetic acid, including treating acetic acid having a poor hue with a synthetic adsorbent to obtain acetic acid with an improved hue.

[2] The method for producing purified acetic acid according to [1], wherein the acetic acid to be treated has an absorbance of 0.01 or greater (or 0.02 or greater, 0.025 or greater, or greater than 0.03) at a wavelength of 430 nm.

[3] The method for producing purified acetic acid according to [1] or [2], wherein the acetic acid to be treated is acetic acid recovered from an acetic acid-containing solution that is discharged in a cellulose acetate production process.

[4] A method for producing purified acetic acid, including:

an extraction step (A) of bringing an acetic acid-containing liquid discharged from a cellulose acetate production process into contact with an extractant to separate the acetic acid-containing liquid into an extraction liquid phase (mainly) containing acetic acid and the extractant and an extraction residual phase (mainly) containing water and soluble cellulose acetate;

an evaporation step (B) of introducing at least a part of the extraction liquid phase into an evaporator for evaporation to separate the extraction liquid phase into vapor containing acetic acid, the extractant, and water and an evaporation residual liquid containing soluble cellulose acetate;

a distillation step (C) of supplying the vapor (or the extraction liquid phase and the vapor) to a distillation column to separate the vapor into a distillate containing the extractant and water and a purified liquid containing acetic acid; and

an adsorption step (D) of bringing the purified liquid containing acetic acid into contact with a synthetic adsorbent to obtain purified acetic acid with an improved hue.

[5] A method for producing purified acetic acid, including:

an extraction step (A) of bringing an acetic acid-containing liquid discharged from a cellulose acetate production process into contact with an extractant to separate the acetic acid-containing liquid into an extraction liquid phase (mainly) containing acetic acid and the extractant and an extraction residual phase (mainly) containing water and soluble cellulose acetate;

a distillation step (C) of supplying the extraction liquid phase to a distillation column to separate the extraction liquid phase into a distillate containing the extractant and water and a purified liquid containing acetic acid; and

an adsorption step (D) of bringing the purified liquid containing acetic acid into contact with a synthetic adsorbent to obtain purified acetic acid with an improved hue.

[6] The method for producing purified acetic acid according to any one of [1] to [5], wherein the synthetic adsorbent is a polystyrene-based synthetic adsorbent or a polymethacrylate-based synthetic adsorbent.

[7] The method for producing purified acetic acid according to [6], wherein the polystyrene-based synthetic adsorbent is at least one selected from a group consisting of (a) a synthetic adsorbent containing a styrene-based polymer as a resin matrix and (b) a synthetic adsorbent containing a styrene-based crosslinked copolymer as a resin matrix.

[8] The method for producing purified acetic acid according to [7], wherein the styrene-based polymer in the (a) synthetic adsorbent containing the styrene-based polymer as the resin matrix is a styrene polymer, an ethylstyrene polymer, or a methylstyrene polymer.

[9] The method for producing purified acetic acid according to [7] or [8], wherein the styrene-based crosslinked copolymer in the (b) synthetic adsorbent containing the styrene-based crosslinked copolymer as the resin matrix is a crosslinked copolymer of at least one styrene-based monomer selected from the group consisting of styrene, ethylstyrene, and methylstyrene and at least one crosslinking agent selected from the group consisting of divinylbenzene and trivinylbenzene.

[10] The method for producing purified acetic acid according to any one of [1] to [9], wherein the synthetic adsorbent has a cation exchange group on a resin surface.

[11] The method for producing purified acetic acid according to any one of [1] to [10], wherein the synthetic adsorbent has a pore structure.

[12] The method for producing purified acetic acid according to [11], wherein an average pore size of the pore structure is 10 nm or less (or 9 nm or less, or 8 nm or less), or 20 nm or greater (or 25 nm or greater, or 30 nm or greater).

[13] The method for producing purified acetic acid according to [11], wherein the pore structure is a binary pore structure.

[14] The method for producing purified acetic acid according to [13], wherein the pore structure is a binary pore structure of small pores having an average pore size of 10 nm or less (e.g., from 2 to 10 nm), and large pores having an average pore size of 20 nm (e.g., from 20 to 100 nm).

[15] The method for producing purified acetic acid according to any one of [1] to [14], wherein a specific surface area of the synthetic adsorbent is from 300 to 1500 m$^2$/g (or from 400 to 1200 m$^2$/g).

[16] The method for producing purified acetic acid according to any one of [1] to [15], wherein a pore volume of the synthetic adsorbent is from 0.1 to 2.0 mL/g (or from 0.2 to 1.7 mL/g).

[17] The method for producing purified acetic acid according to any one of [1] to [16], wherein the synthetic adsorbent has a spherical shape having an average particle size from 200 to 1500 $\mu$m (or from 300 to 1200 $\mu$m).

Industrial Applicability

According to the method for producing purified acetic acid according to an embodiment of the present disclosure, purified acetic acid with a good hue is efficiently obtained.

Reference Signs List

1: Extraction column
2: Evaporator
2a, 3a: Reboiler
3: Distillation column
4: Adsorption apparatus
11 to 22: Line

**Claims**

1. A method for producing purified acetic acid, comprising treating acetic acid having a poor hue with a synthetic adsorbent to obtain acetic acid with an improved hue.

2. The method for producing purified acetic acid according to claim 1, wherein the acetic acid to be treated has an absorbance of 0.01 or greater at a wavelength of 430 nm.

3. The method for producing purified acetic acid according to claim 1 or 2, wherein the acetic acid to be treated is acetic acid recovered from an acetic acid-containing solution that is discharged in a cellulose acetate production process.

4. The method for producing purified acetic acid according to any one of claims 1 to 3, wherein the synthetic adsorbent has a cation exchange group on a resin surface.

5. The method for producing purified acetic acid according to any one of claims 1 to 4, wherein the synthetic adsorbent has a pore structure.

6. The method for producing purified acetic acid according to claim 5, wherein the pore structure is a binary pore structure.

FIG. 1

| | | |
|---|---|---|
| **INTERNATIONAL SEARCH REPORT** | International application No. | |
| | PCT/JP2021/011666 | |

**A. CLASSIFICATION OF SUBJECT MATTER**
Int.Cl. C07C51/47(2006.01)i, C07C53/08(2006.01)i
FI: C07C51/47, C07C53/08

According to International Patent Classification (IPC) or to both national classification and IPC

**B. FIELDS SEARCHED**

Minimum documentation searched (classification system followed by classification symbols)
Int.Cl. C07C51/47, C07C53/08

Documentation searched other than minimum documentation to the extent that such documents are included in the fields searched
    Published examined utility model applications of Japan    1922-1996
    Published unexamined utility model applications of Japan    1971-2021
    Registered utility model specifications of Japan    1996-2021
    Published registered utility model applications of Japan    1994-2021

Electronic data base consulted during the international search (name of data base and, where practicable, search terms used)
    JSTPlus/JMEDPlus/JST7580(JDreamIII), CAplus/REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 64-9950 A (WAKO PURE CHEMICAL INDUSTRIES, LTD.) 13 January 1989 (1989-01-13), page 303, right column, line 6 to page 304, upper right column, line 15, page 304, upper right column, lines 12-15, examples | 1, 2, 4-6<br>1-6 |
| Y | JP 2011-515468 A (CELANESE INTERNATL CORP.) 19 May 2011 (2011-05-19), paragraph [0004] | 1-6 |
| A | JP 2018-119052 A (DAICEL CORPORATION) 02 August 2018 (2018-08-02) | 1-6 |

☐ Further documents are listed in the continuation of Box C.     ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 May 2021 | 25 May 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.

PCT/JP2021/011666

| | | |
|---|---|---|
| JP 64-9950 A | 13 January 1989 | (Family: none) |
| JP 2011-515468 A | 19 May 2011 | US 2009/0247788 A1<br>paragraph [0003]<br>WO 2009/120257 A1<br>EP 2271610 A1<br>KR 10-2010-0132987 A<br>CN 101980998 A |
| JP 2018-119052 A | 02 August 2018 | US 2019/0389977 A1<br>WO 2018/139319 A1<br>EP 3575329 A1<br>CN 110248965 A |

Form PCT/ISA/210 (patent family annex) (January 2015)

**EP 4 129 970 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020056079 A
- JP 59007131 A
- JP 60174743 A